Europäisches Patentamt

European Patent Office

Office européen des brevets

(11) Veröffentlichungsnummer: **0 207 299**
**B1**

(12) **EUROPÄISCHE PATENTSCHRIFT**

(45) Veröffentlichungstag der Patentschrift:
**12.04.89**

(51) Int. Cl.⁴: **A61F 2/36**

(21) Anmeldenummer: **86107390.6**

(22) Anmeldetag: **30.05.86**

(54) Femurhüftgelenk-Endoprothese.

(30) Priorität: **25.06.85 DE 3522692**

(43) Veröffentlichungstag der Anmeldung:
**07.01.87 Patentblatt 87/2**

(45) Bekanntmachung des Hinweises auf die Patenterteilung:
**12.04.89 Patentblatt 89/15**

(84) Benannte Vertragsstaaten:
**AT BE CH DE FR GB IT LI NL SE**

(56) Entgegenhaltungen:
**EP-A- 0 093 230**
**DE-A- 2 724 234**
**DE-A- 2 832 555**
**DE-B- 2 555 717**

**ARCHIV FÜR ORTHOPÄDISCHE UND UNFALL CHIRURGIE, Band 86, 1976, Seiten 1-14; G. RITTER et al.: "Die Zuggurtungs-Hüftendoprothese"**

(73) Patentinhaber: **S + G IMPLANTS GMBH,**
**Grapengiesserstrasse 21, D-2400 Lübeck(DE)**

(72) Erfinder: **Ritter, Gebhard, Prof., Jupiterweg 13,**
**D-6500 Mainz-Fürthen(DE)**
Erfinder: **Grundei, Hans, Gärtnergasse 4,**
**D-2400 Lübeck(DE)**

(74) Vertreter: **Wilcken, Thomas, Dipl.-Ing. et al,**
**Musterbahn 1, D-2400 Lübeck(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Femurhüftgelenk-Endoprothese, deren gekrümmter Schaft mit einem sich auf eine Knochenresektionsfläche abstützenden Prothesenkragen mit einem die aufsetzbare Gelenkkugel tragenden Klemmkonuszapfen versehen ist, wobei eine an die Prothese angreifende anziehbare Schraube als Zuggurt eine von der Resektionsfläche ausgehende laterale, schräg nach unten und außen gerichtete Bohrung des Femurknochens durchläuft.

Es ist bekannt, daß Hüftgelenk-Endoprothesen bei der Verankerung des die Gelenkkugel tragenden Schaftes durch das Gewicht des Patienten exzentrisch belastet werden, so daß dadurch mit der Zeit Prothesenlockerungen oder sogar durch Biegebeanspruchung Prothesenbrüche auftreten können und auch aufgetreten sind, da die hohe Druckbelastung über den Prothesenkragen auf den medialen Teil des Röhrenknochens übertragen werden. Um hierzu gegenüber den sich medial auswirkenden Druckkräften einen Ausgleich zu schaffen, ist man so vorgegangen, daß der Prothesenkragen lateral speziell ausgebildet wurde, damit er von einer Schraube durchgriffen werden kann, die eine von der Resektionsfläche schräg nach unten und außen gerichtete Durchbohrung des Knochens durchläuft und durch eine aufschraubbare Mutter angezogen wird, so daß die medialen Druckkräfte durch laterale Zugkräfte ausgeglichen werden (siehe den Artikel "Die Zuggurtungs-Hüftendoprothese" von G. Ritter, A. Grünert und C. H. Schweikert, abgedruckt im "Archiv für orthopädische und Unfall-Chirurgie 1976).

Heute werden Femurhüftgelenk-Endoprothesen in vielen Fällen so ausgebildet, daß der Prothesenkragen mit einem Klemmkonus zum Aufsetzen der Gelenkkugel mit Reibungsschluß erfolgt, so daß dadurch eine bessere Anpassung der Endoprothesen an verschiedene Hüftgelenke von Patienten erfolgen kann.

Die Aufgabe der Erfindung besteht darin, bei solchen Prothesen spezielle Ausbildungen des Prothesenkragens und dessen Durchbohrung zu vermeiden, aber trotzdem eine Möglichkeit zu schaffen, eine Verbindung der durch eine Knochenbohrung verlaufenden Zugschraube mit der Prothese herzustellen.

Diese Aufgabe wird bei der eingangs erwähnten Femurhüftgelenk-Endoprothese nach der Erfindung dadurch gelöst, daß auf den Konuszapfen ein vorteilhaft zylindrischer Ring form- und kraftschlüssig aufschiebbar ist, der einen lateralen, den Prothesenkragen übergreifenden, flächenförmigen Ausleger aufweist, dessen freies, die Resektionsfläche berührendes Ende eine mit der Knochenbohrung fluchtende Durchbohrung für den Durchgriff einer anziehbaren Schraube aufweist.

Es kann nunmehr auf besonders einfache und bequeme Weise auf den Klemmkonus ein vorteilhafter zylindrischer Ring mit dem Ausleger bis an den Prothesenkragen heran aufgeschoben werden, ohne daß eine Änderung der üblichen Femurhüftgelenk-Endoprothesen erfolgen muß. Es ist lediglich erforderlich, für unterschiedliche Größen der Endoprothese einige angepaßte Ringgrößen mit Ausleger vorrätig zu halten.

Die Erfindung wird nachstehend anhand der Zeichnungen beschrieben, in der ein Ausführungsbeispiel schematisch dargestellt ist. Es zeigen:

Figur 1 den Oberteil einer Femurhüftgelenk-Endoprothese in Seitenansicht und deren Implantation in den Femur-Röhrenknochen entsprechend der Erfindung,

Figur 2 eine Aufsicht auf den auf den Klemmkonus aufschiebbaren Ring mit Ausleger,

Figur 3 einen Schnitt nach Linie III–III der Figur 2.

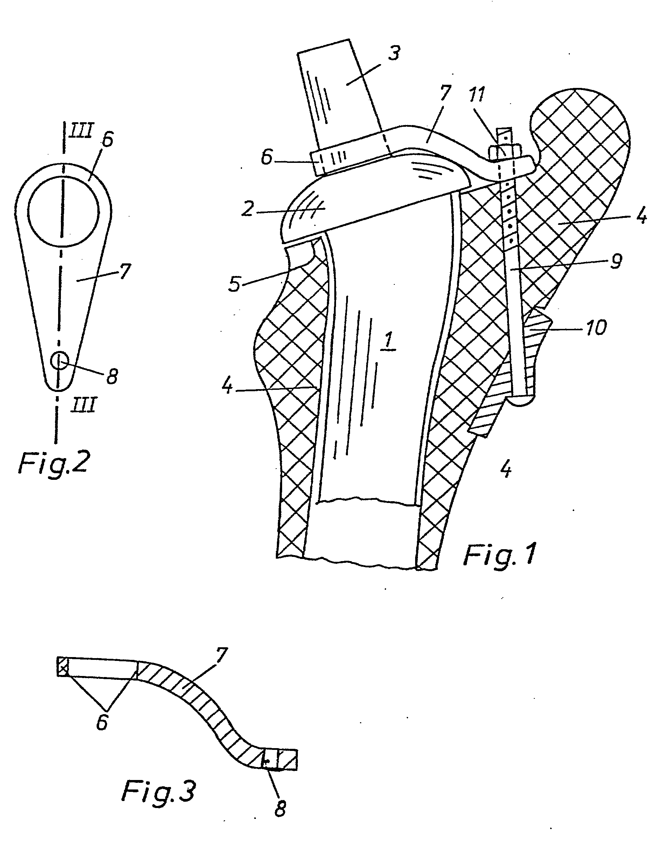

Die Femurhüftgelenk-Endoprothese nach Figur 1 besteht in bekannter Weise aus einem nur teilweise dargestellten, gekrümmten Schaft 1 mit Prothesenkragen 2 und einem unmittelbar am Kragen zylindrischen Klemmkonus 3 zum Aufsetzen einer Gelenkkugel. Zur Implantation wird der Schenkelhals des Femur-Röhrenknochens durchtrennt, möglichst derart, daß die Resektionsfläche 5 einen Winkel von 65° zum Schaft 1 bildet.

Entsprechend der Erfindung wird zum Ausgleich der an der implantierten Prothese angreifenden, durch das Gewicht des Patienten auftretenden Druckkräfte ein Ring, vorteilhaft Zylinderring 6, auf den Klemmkonus 3 kraft- und formschlüssig bis zum Prothesenkragen 2 aufgeschoben. Dieser Zylinderring 6 besitzt einen flächenförmigen, lateral gerichteten Ausleger 7, der den Prothesenkragen 2 bogenförmig übergreift und mit seinem, eine Durchbohrung aufweisenden Ende an der Resektionsfläche 5 anliegt.

Vor der Implantation der Femurhüftgelenk-Endoprothese wird der Femurknochen lateral, ausgehend von der Resektionsfläche 5, mit einer schräg nach unten und außen gerichteten Durchbohrung versehen. Nach der Implantation der Endoprothese wird durch die Knochenbohrung und die Auslegerbohrung 8 eine Schraube 9 gesteckt, die sich mit einem Ende an einer äußeren Platte 10 abstützt, die in gewünschten Fällen geringfügig im Knochen 4 versenkt ist, während auf dem anderen Ende eine Mutter 11 aufschraubbar ist, durch die der Ausleger 7 und damit die Endoprothese fest mit dem Knochen über die Resektionsfläche verbunden wird. Durch diese Verbindung der implantierten Endoprothese mit dem Knochen 4 werden die medial durch das Körpergewicht des Patienten auf den Femurknochen ausgeübten Druckkräfte durch laterale Zugkräfte ausgeglichen.

## Patentansprüche

Femurhüftgelenk-Endoprothese, deren gekrümmter Schaft (1) mit einem sich auf eine Knochenresektionsfläche abstützenden Prothesenkragen (2) mit einem die aufsetzbare Gelenkkugel tragenden Klemmkonus (3) versehen ist, wobei eine an die Prothese angreifende anziehbare Schraube (9) als Zuggurt eine von der Resektionsfläche ausgehende laterale, schräg nach unten und außen gerichtete Bohrung des Femurknochens durchläuft, dadurch gekennzeichnet, daß auf den Konuszapfen (3) ein vorteilhaft zylindrischer Ring (6) form- und kraftschlüssig aufschiebbar ist, der einen lateralen, den Prothesenkragen (2) übergreifenden, flächenförmigen Ausleger (7) aufweist, dessen freies, die Resektionsfläche (5) berührendes Ende eine mit der Knochenbohrung fluchtende Durchbohrung (8) für den Durchgriff einer anziehbaren Schraube (9) aufweist.

## Claims

Femur hip joint endoprosthesis, the curved shank (1) of which, is provided with a prosthesis collar (2) bearing on a bone surgical removal surface and comprising a grippable cone (3) carrying the attachable joint ball, a tightenable bolt (9) acting on the prosthesis as a tension strap traversing a lateral bore of the femur bone starting from the surgical removal surface and directed obliquely downwards and outwards, characterised in that an advantageously cylindrical ring (6) may be pushed on to the tapered pin (3) in a shape and force determined manner, which ring has a lateral surface-forming arm (7) extending over the prosthesis collar (2), the free extremity of which touching the surgical removal surface (5) has a bore (8) in alignment with the bone bore for passing through by a tightenable bolt (9).

## Revendications

Endoprothèse fémorale d'articulation de la hanche, dont la broche cintrée (1) est pourvue d'un col (2) formé par la prothèse, qui prend appui sur une surface de résection de l'os et possède un cône de serrage portant la boule d'articulation pouvant être emmanchée sur ce cône, et dans laquelle un boulon (9) apte à être serré et accroché à la prothèse, traverse, en tant qu'élément de traction, un perçage latéral du fémur, que s'étend à partir de la surface de résection en étant dirigé obliquement vers le bas et vers l'extérieur, caractérisée en ce que sur le tenon conique (3) il est possible d'emmancher, conformément à une liaison par formes complémentaires et à une liaison de force, un anneau (6) avantageusement cylindrique, qui possède un bras latéral (7), d'une certaine étendue en surface, s'engageant par-dessus le col (2) de la prothèse et dont l'extrémité libre, qui est en contact avec la surface de résection (5), possède un passage traversant (8) aligné avec le perçage ménagé dans l'os et permettant le passage d'un boulon (9) pouvant être serré.

Fig.2

Fig.1

Fig.3